# FASCICULE DE BREVET EUROPEEN

(11) **EP 0 883 406 B1**
(45) Date de publication et mention de la délivrance du brevet: **14.04.2004**
(21) Numéro de dépôt: 97907134.7
(22) Date de dépôt: 26.02.1997
(51) Int. Cl.: A61K 38/20, A61K 35/14

(54) **UTILISATION DE L'IL-7 DANS LE TRAITEMENT DES MALADIES AUTO-IMMUNES, EN PARTICULIER LE DIABETE MELLITUS INSULINODEPENDANT**
VERWENDUNG VON IL-7 ZUR BEHANDLUNG VON AUTOIMMUNKRANKHEITEN, INSBESONDERE INSULINABHÄNGIGEM DIABETES MELLITUS
USE OF IL-7 FOR TREATING AUTO-IMMUNE DISEASES AND INSULIN-DEPENDENT DIABETES MELLITUS IN PARTICULAR

(30) Priorité: 28.02.1996 FR 9602501
(43) Date de publication de la demande: 16.12.1998
(73) Titulaire: SANOFI-SYNTHELABO, 75635 Paris Cédex 13 (FR)
(72) Inventeur: BACH, Jean-François, F-75007 Paris (FR); GOMBERT, Jean-Marc, F-86000 Poitiers (FR); HERBELIN, André, F-92240 Malakoff (FR); MORRE, Michel, F-92100 Boulogne (FR)
(74) Mandataire: Bernasconi, Jean Raymond
(86) Numéro de dépôt international: PCT/FR1997/000343
(87) Numéro de publication internationale: WO 1997/031648

(56) Documents cités:
- WO-A-92/01459
- WO-A-96/01122
- FASEB JOURNAL, vol. 4, no. 7, 1990, page A2183 XP002019544 LYNCH ET AL: "IL-7 INDUCES LAK ACTIVITY IN RESTING MURINE T CELLS"
- DATABASE MEDLINE FILE SERVER STN KARLSRUHE ABR G 94101260, COSTELLO ET AL: "THE PLEIOTROPIC EFFECTS OF INTERLEUKIN 7 AND THEIR PATHOLOGIC AND THERAPEUTIC IMPLICATIONS" XP002019546
- IMMUNOLOGY, vol. 80, no. 3, 1 Novembre 1993, pages 451-457, XP000560404 COSTELLO R ET AL: "INTERLEUKIN-7 IS A POTENT CO-STIMULUS OF THE ADHESION PATHWAY INVOLVING CD2 AND CD28 MOLECULES"
- J.IMMUNOL., vol. 155, 1995, pages 4544-4550, XP002019545 DO CARMO LEITE-DE-MORAES ET AL: "MHC CLASS I-SELECTED CD4-CD8-TCR-ALPHABETA+ T CELLS ARE A POTENTIAL SOURCE OF IL-4 DURING PRIMARY IMMUNE RESPONSE" cité dans la demande
- J.EXP.MED., vol. 178, 1993, pages 87-99, XP000610471 RAPOPORT ET AL: "INTERLEUKIN 4 REVERSES T CELL PROLIFERATIVE UNRESPONSIVENESS AND PREVENTS THE ONSET OF DIABETES IN NONOBESE DIABETIC MICE" cité dans la demande
- J.EXP.MED., vol. 178, 1993, pages 901-908, XP000611517 BIX ET AL: "POSITIVE SELECTION OF VBETA8+CD4-8- THYMOCYTES BY CLASS I MOLECULES EXPRESSED BY HEMATOPOIETIC CELLS" cité dans la demande
- J.EXP.MED., vol. 180, 1994, pages 653-661, XP000611515 VICARI ET AL: " INTERLEUKIN 7 INDUCES PREFERENTIAL EXPANSION OF VBETA8.2+CD4-8- AND VBETA8.2+CD4+8- MURINE THYMOCYTES POSITIVELY SELECTED BY CLASS I MOLECULES" cité dans la demande
- AUTOIMMUNITY, vol. 15, 1993, pages 113-122, XP000610469 ANDERSON ET AL: "INSULIN-DEPENDENT DIABETES IN THE NOD MOUSE MODEL II. BETA CELL DESTRUCTION IN AUTOIMMUNE DIABETES IS A TH2 AND NOT A TH1 MEDIATED EVENT" cité dans la demande
- C.R.ACAD.SCI.PARIS,SCIENCES DE LA VIE/LIFE SCIENCES, vol. 319, Février 1996, pages 125-129, XP000608939 GOMBERT ET AL: "EARLY DEFECT OF IMMUNOREGULATORY T CELLS IN AUTOIMMUNE DIABETES"
- INTERNATIONAL IMMUNOLOGY, vol. 8, no. 11, Novembre 1996, pages 1751-1758, XP000674709 GOMBERT ET AL: "IL-7 REVERSES NK1+ T CELL-DEFECTIVE IL-4 PRODUCTION IN THE NON-OBESE DIABETIC MOUSE"

## Description

La présente invention concerne une nouvelle utilisation de l'interleukine-7 (IL-7) dans le traitement des maladies auto-immunes, en particulier le diabète mellitus insulinodépendant.

Le diabète de type 1 ou diabète mellitus insulinodépendant (DMID) est considéré aujourd'hui comme une maladie auto-immune (Endocr. Rev. 1994, *15* (4), 516-542), caractérisée par la présence d'anticorps anti-cellules béta et par sa sensibilité à une thérapie immunosuppressive. Le DMID résulte à la fois chez l'homme et chez la souris "nonobese diabetic" (NOD), d'une réponse immunitaire de type cellulaire prédominante, la réponse humorale étant caractérisée par la sécrétion d'anticorps anti-membrane et anti-produits sécrétoires de cellules béta (N. Engl. J. Med. 1981, *304*, 1454-1465, N. Engl. J. Med., 1992, *327*, 302). La réponse immunitaire de type cellulaire est caractérisée par des lésions histologiques ou "insulitis" provoquées par des infiltrations de cellules inflammatoires et immunitaires de types macrophages, lymphocytes B et T à l'intérieur des ilots de Langerhans du pancréas (Diabetologia, 1989, 32, 282-289 ; Insulitis and type 1 diabetes, Academic Press Tokyo, 1986, 35-50).

La souns NOD est un modèle spontané du diabète auto-immun ou diabète de type 1. Des arguments convergents indiquent que la survenue de la maladie est sous le contrôle de cellules T immunorégulatnces CD4⁺. Celle-ci est en effet accélérée par la thymectomie réalisée à l'âge de 3 semaines (Eur. J. Immuno, 1989, *19*, 889-895) et peut être prévenue, dans un système de transfert, par la coinjection de thymocytes ou de cellules spléniques CD4⁺ de jeunes souris NOD non encore diabétiques (J. Exp. Med., 1989, *169*, 1669-1680).

Il a été suggéré et montré de manière indirecte que les cellules lymphocytes T helper 2 (Th2) de par leur capacité à produire de l'interteukine-4 (IL-4) sont les cellules T immunorégulatrices CD4⁺ dont il est question (Autoimmunity, 1993, *15*, 113-122). Il a en effet été montré que l'administration d'IL-4 (J. Exp. Med. 1993, *178* (1), 87-99) ou d'anticorps monoclonal anti-interféron-γ (IFN-γ) prévient le déclenchement du diabète et que l'administration d'interleukine-12 (IL-12), inducteur de lymphocytes T helper 1 (Th1), accélère le déclenchement du diabète (J. Exp. Med., 1995, *181* (2), 817-821).

Néanmoins, une autre étude a montré que si des cellules "Th2-like" diabétologiques retrouvées infiltrées dans les îlots ne provoquaient pas l'apparition de la maladie, elles n'offraient pas pour autant de protection significative (Science, 1995, 268 (5214), 1185-1188). Ainsi, s'il a été suggéré et montré de manière indirecte que le déclenchement du diabète chez la souris NOD est sous le contrôle des cellules Th2, aucune explication n'est donnée ou suggérée sur le caractère de l'anomalie présente chez les souris NOD, du processus physiologique qui en découle et qui est à l'origine de l'émergence d'une auto-immunité anti-îtots de Langherans à l'origine du diabète chez ces animaux. Il a également été suggéré récemment que la différenciation des cellules Th2 pouvait être contrôlée par un sous-type de cellules T caractérisé par le phénotype TCR-αβ, CD4⁻CD8⁻(double négative, DN) ou CD4⁺CD8⁻(positive simple), porteurs d'un phénotype mature (non expression du "Heat Stable Antigen", HSA), exprimant sélectivement le marqueur d'activation CD44. Cette sous-population, qui est caractérisée également dans d'autres souches par le marqueur NK1.1, a la capacité de produire en grande quantité de l'IL-4 après stimulation par un anticorps polyclonal anti-TCR-αβ (TCR-αβ : récepteur αβ des cellules T) (J. Immunol. 1995, *155* (10), 4544-4550).

D'autre part, il a été montré que ce sous-type a la particularité d'être restreint par des molécules de classe I du complexe majeur d'histocompatibilité (CMH) et d'utiliser préférentiellement le gène Vβ8 du récepteur T (J. Exp. Med., 1993, *178*, 901-908), sous type dont la prolifération est induite de manière spécifique par l'interieukine-7 (J. Exp. Med. 1994, *180* (2), 653-661).

Les protéines interieukine-7 de mammifères (cytokine IL-7s), en particulier chez l'homme et la souris, les ADNs correspondants, les vecteurs d'expression codant pour les IL-7s, leurs procédés de production, incluant les systèmes recombinants ont été décrits (US 4 965 195).

Les protéines interieukine-7 de mammifères sont désignées ci-après "IL-7". L'IL-7 est un facteur de croissance lymphopoiétique qui a la capacité de stimuler le développement et la prolifération de cellules de la moelle osseuse (WO 89/03884). La stimulation de la production de plaquettes (WO 90/09194) par induction de la prolifération de mégacaryocytes a été une des premières applications de l'administration de l'IL-7. D'autres applications de l'IL-7 ont également été décrites comme celle pour le traitement du cancer ou de l'infection virale par immunothérapie à partir de cellules modifiées produisant de l'IL-7, soit par une injection directe de cellules modifiées *in vivo*, soit par une phase préalable de traitement *in vitro* avant injection (WO 92/01459). A également été décrite l'utilisation de l'IL-7 pour la prolifération de lymphocytes T humains spécifiques anti-HIV comme thérapie potentielle du SIDA (J. of Leucocyte Biology, 1995, *58* (6), 623-633), pour le traitement du mélanome malin dans le domaine de la dermatologie (Hautarzt, 1995, *46* (10), 676-682) ainsi que comme potentialisateur d'un vaccin pour la prévention d'une infection (microbienne et virale) et de l'établissement d'une tumeur (WO 94/22473). A également été décrite l'utilisation d'anticorps anti-IL-7 pour l'étude et la recherche de processus physiologiques et pathologiques, en particulier concernant la différenciation et la prolifération des lymphocytes (WO 94/28160). D'autres applications associant l'utilisation de l'IL-7 avec d'autres cytokines ont été décrites comme l'association avec l'IL-4 pour l'induction *in vitro* de la différenciation des cellules pré-B (WO 94/04658) ou avec l'IL-3 pour le traitement de la leucopénie (WO 92/04455) et pour la prévention de désordre de la moelle osseuse après thérapie cancéreuse ou greffe de moelle (WO 93/03061).

Lynch et al., FASEB vol. 4, no 7, 1990, page A2183 décrivent l'immunothérapie des tumeurs utilisant des lymphocytes T stimulés par l'IL-7.

WO 96/01122 décrit l'induction d'une réponse immune contre un pathogène ou une tumeur à l'aide de lymphocytes T stimulés *ex vivo* avec l'IL-7 (page 2, lignes 16-31) et le traitement des maladies auto-immunes avec des lymphocytes T maintenus non-réactifs en les cultivant *ex vivo* avec un antigène et un anticorps anti-IL-7, inhibant le signal cellulaire normalement induit par l'IL-7 sur la chaîne du récepteur de cytokines (page 2, ligne 32 - page 3, ligne 6).

Les auteurs de la présente invention ont à présent montré sur la base d'une étude phénotypique en cytométrie de fiux utilisant les marqueurs HSA, CD4, CD8, CD44 et Vβ8, qu'une sous population de thymocytes de phénotype CD44⁺TCR-αβ HSA⁻ et utilisant préférentiellement le gène Vβ8 du récepteur T est numériquement réduite chez la souris NOD âgée de 3 semaines (à la fois pour les populations CD4⁻CD8⁻DN (double négative CD4⁻CD8⁻) et CD4⁺ (simple positive)) et âgée de 8 semaines (pour la population DN). Il a été également trouvé qu'aux deux âges, cette sous-population produit peu ou pas d'IL-4. Enfin, les auteurs de la présente invention ont montré que cette double anomalie numérique et fonctionnelle est corrigée *in vitro* et *in vivo* par l'IL-7, facteur de croissance de cette sous-population cellulaire et qu'ainsi l'IL-7 est en mesure de protéger des mammifères du diabète, notamment du diabète mellitus insulinodépendant, cette propriété pouvant s'étendre également à toutes maladies auto-immunes, générées par un défaut de production d'IL-4 par les cellules Th2 et notamment par ladite sous population cellulaire décrite ci-dessus et de manière générale à toutes maladies auto-immunes, notamment celles générées par un défaut dans l'immunorégulation par les cellules CD4⁺.

Ainsi, selon l'un de ses aspects, la présente invention a pour objet l'utilisation de l'IL-7 ou de lymphocytes T préalablement incubés en présence d'IL-7 ou modifiés produisant l'IL-7, pour la préparation de médicaments ou compositions pharmaceutiques pour le traitement des maladies auto-immunes, en particulier les maladies auto-immunes générées par un défaut dans l'immunorégulation par les cellules T CD4⁺.

Sont préférées parmi lesdites maladies auto-immunes, les maladies auto-immunes qui sont générées par un défaut de production d'IL-4 par les cellules Th2.

Tout particulièrement préférées parmi lesdites maladies auto-immunes sont les maladies auto-immunes générées par un défaut de production d'IL-4 lié à un déficit quantitatif et fonctionnel de sous-type de cellules T de phénotype HSA⁻, CD4⁻CD8⁻ ou CD4⁺CD8⁻, CD44⁺, TCR-αβ⁺, Vβ8⁺, NK 1.1⁺

Parmi lesdites maladies auto-immunes, outre le diabète mellitus insulinodépendant, l'encéphalo-myélite auto-immune, l'arthnte rhumatoïde auto-immune, la polyarthrite, les hépatites auto-immunes de type 2, la gastrite auto-immune, la sclérose auto-immmune, la sialadénite, l'adrénalite, l'oophorite, les glomérulonéphrites, la thyroïdite auto-immune peuvent préférentiellement être traitées par lesdits médicaments ou compositions, comme tout mécanisme pathogénique de type auto-immun dans une thérapie associée au traitement du SIDA.

Préférentiellement, la maladie auto-immune est le diabète mellitus insulinodépendant.

Avantageusement, les lymphocytes T préalablement incubés en présence d'IL-7, utilisés selon la présente invention, sont des cellules autologues ou syngéniques des cellules des patients auxquels les compositions les comprenant sont destinées.

L'invention comprend également des compositions pharmaceutiques offrant une nouvelle approche pour traiter les maladies auto-immunes générées par un défaut dans l'immunorégulation par les cellules T CD4⁺.

En particulier l'invention comprend des compositions pharmaceutiques offrant une nouvelle approche pour traiter les maladies auto-immunes générées par un défaut de production d'IL-4 par les cellules Th2, tout particulièrement les maladies auto-immunes générées par un défaut de production d'IL-4 lié à un déficit quantitatif et fonctionnel de sous-type de cellules T de phénotype HSA⁻, CD4⁻CD8⁻ ou CD4⁺CD8⁻, CD44⁺, TCR-αβ⁺, Vβ8⁺, NK 1 1⁺ comme notamment le diabète mellitus insulinodépendant.

De telles compositions comprennent comme principe actif de l'IL-7, préférentiellement sous forme soluble, et/ou des lymphocytes T autologues ou syngéniques des cellules du patient auquel la composition pharmaceutique est destinée, lesdits lymphocytes T ayant été préalablement incubés en présence d'IL-7. Elles peuvent également se présenter sous forme d'associations avec d'autres principes actifs, par exemple d'autres agents immunomodulateurs.

Ces différentes compositions peuvent être administrées selon plusieurs modes d'administration, que l'homme du métier pourra déterminer en fonction du type de composition concernée.

Les compositions comprenant de l'IL-7 comme principe actif peuvent par exemple être administrées par voie systémique, de préférence par voie intraveineuse, par voie intramusculaire, intradermique ou par voie orale.

Les compositions comprenant des lymphocytes T comme principe actif sont préférentiellement administrées par voie intraveineuse ou intrapéritonéale.

Les modes d'administration préférés ainsi que les posologies et formes galéniques optimales peuvent être déterminés selon les critères généralement pris en compte dans l'établissement d'un traitement thérapeutique adapté à un patient comme par exemple l'âge ou le poids corporel du patient, la gravité de son état général, la tolérance au traitement et les effets secondaires constatés.

La présente invention concerne également un procédé de fabrication d'un médicament ou composition pharmaceutique pour le traitement des maladies auto-immunes, en particulier les maladies auto-immunes générées par un défaut dans l'immunorégulation par les cellules T CD4⁺ caractérisé en ce que l'on mélange de l'IL-7 et/ou des lymphocytes T autologues ou syngéniques des cellules du patient auquel la composition est destinée, lesdits lymphocytes T ayant été préalablement incubés en présence d'IL-7, avec un véhicule ou diluant pharmaceutiquement acceptable, éventuellement en association avec d'autres principes actifs.

En particulier l'invention concerne un procédé de fabrication d'un médicament
ou composition pharmaceutique pour le traitement de maladies auto-immunes générées par un défaut de production d'IL-4 par les cellules Th2, tout particulièrement les maladies auto-immunes générées par un défaut de production d'IL-4 lié à un déficit quantitatif et fonctionnel de sous-type de cellules T de phénotype HSA⁻, CD4⁻CD8⁻ ou CD4⁺CD8⁻, CD44⁺, TCR-αβ⁺, Vβ8⁺, NK1.1⁺ comme notamment le diabète mellitus insulinodépendant.

La présente invention concerne une méthode de traitement thérapeutique caractérisée en ce que l'on administre une dose thérapeutiquement efficace d'interteukine-7 ou de lymphocytes T préalablement incubés en présence d'IL-7 à un patient atteint d'une maladie auto-immune, en particulier une maladie auto-immune générée par un défaut dans l'immunorégulation par les cellules T CD4⁺.

De manière avantageuse, la méthode selon l'invention s'applique au traitement du diabète mellitus insulinodépendant.

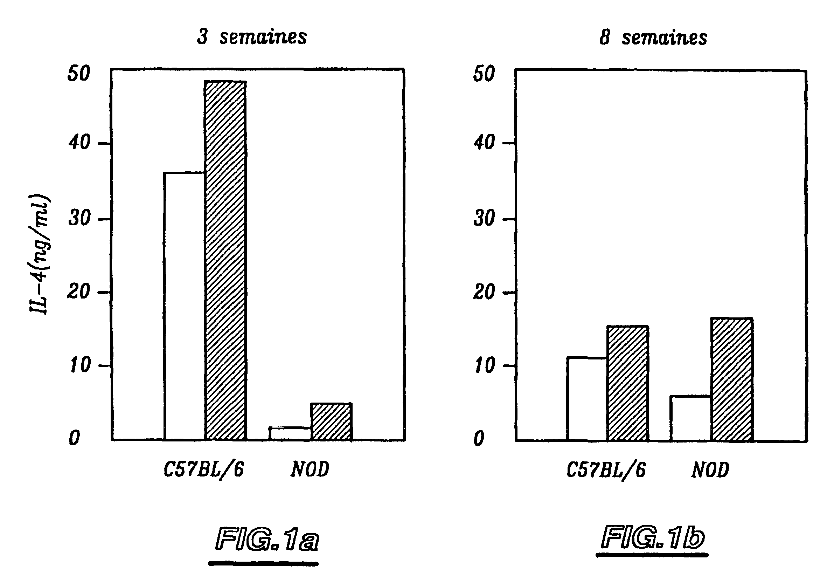

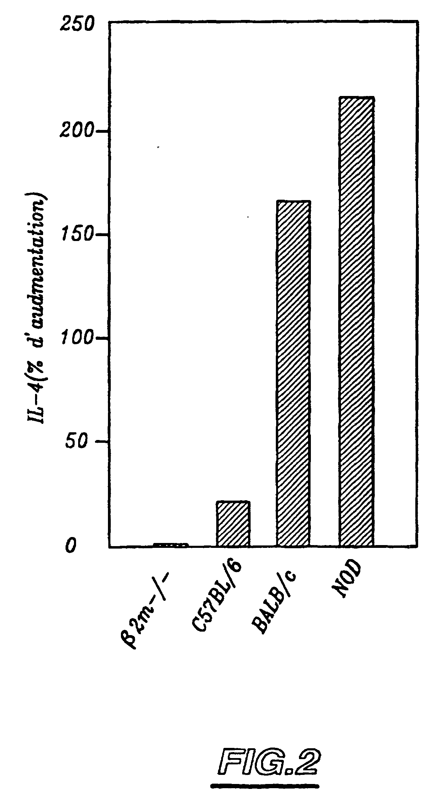

La présente invention est illustrée par les figures 1 et 2 ci-après.

La figure 1 montre l'effet correcteur *in vitro* de l'IL-7 sur la production d'IL-4 par des cellules thymocytes matures HSA⁻ CD8⁻ chez la souris NOD et C57BL/6 jeune (figure 1a) et adulte (figure 1 b).

Dans l'expérience présentée, les thymocytes HSA⁻ CD8⁻ fraîchement recueillis de souris NOD ou C57BL/6 jeunes (âgées de 3 semaines) ou adultes (âgées de 8 semaines) sont cultivés à 1,0 x 10⁵ cellules/puits avec un anticorps monoclonal anti-TCR-αβ en présence (colonne hachurée) ou en absence (colonne vide) d'IL-7 à 1000 U/ml. Le surnageant est recueilli après 48 heures d'incubation et dosé en IL-4 par ELISA (moyenne sur 3 à 4 expériences indépendantes).

D'autres caractéristiques et avantages de l'invention apparaissent dans la suite de la description avec les exemples et les tableaux regroupant les résultats des essais expérimentaux.

La figure 2 montre l'effet du traitement des souris par L'IL-7 sur la production primaire d'IL-4 par les splénocytes.

Dans l'expérience présentée les souris de différentes souches âgées de 8 à 12 semaines reçoivent des injections sous-cutanées quotidiennes de 2µg d'IL-7 ou d'albumine sérique bovine (excipient) pendant 7 jours consécutifs. Le huitième jour, les souris sont traitées par l'anticorps anti-CD3 puis les splénocytes cultivés (5.10⁶ cellules par puits) et la production d'IL-4 mesurée par le test CT.4S. Le pourcentage d'augmentation de la production d'IL-4 est calculé de la manière suivante : ((quantité d'IL-4 produite après traitement par l'IL-7) / (quantité d'IL-4 produite après traitement par l'excipient)-1)x100. Les résultats correspondent à une expérience type. Les productions d'IL-4 (U/ml) par les splénocytes des souris traitées par l'excipient sont respectivement de 94,3 ; 53,5 ; 11,0 et 11,4 pour les souris BALB/c, C57BL/6, NOD et C57BL/6β2m⁺.

### EXEMPLE 1

### Etude de l'ontogénie des thymocytes CD44⁺ TCR-αβ⁻ chez la souris NOD comparée à la souris C57BL/6 non diabétique.

Des souris d'élevage et C57BL/6 femelles sont maintenues dans des conditions d'environnement spécifiques de non pathogénicité. Toutes les souns NOD sont vérifiées comme ne manifestant aucun signe évident de diabète (pas de glycosurie ou d'hyperglycémie). Les thymus d'environ 5 à 15 souns exsangues sont prélevés avec soin puis sont mélangés. Les thymocytes matures double négatifs (ON) et CD4⁺ simples positifs sont ensuite enrichis par incubation à 37°C pendant 40 minutes avec des anticorps anti-CD8 (3-155 ; IgM de rat décrit dans J. Immunol., 1980, *125*, 2665-2672), anti-HSA (J11d; IgM de rat, Pharmigen) et anti-complément (complément de lapin Low Tox, Cederlane, Ontario, Canada). Les cellules mortes sont ensuite éliminées par centrifugation en gradient de densité (J. Prep., Techgen, Les Ulis, France). Une cytométrie de flux des anticorps spécifiques différents de ceux utilisés pour la lyse cellulaire montrent que plus de 95 % des cellules récupérées sont de phénotype HSA⁻ CD8⁻.

Le marquage est effectué comme décrit dans J. Exp. Med. 1994, *180*, 653-661. Les anticorps utilisés sont produits par des hybridomes commercialisés. Les anticorps anti-TCR-αβ (clone H57-597, Pharmigen) ou antiVβ8 (clone F23.1 décrit dans J. Immunol. *143*, 3994-4000) biotinylés ou marqués à la fluorescéine (FITC) sont utilisés combinés avec des anticorps anti-CD4 (clone RM 4.5, Pharmigen) marqués à la Phycoérytrine (PE) et/ou avec des anticorps anti-CD44 (clone IM 7.8, Pharmigen) marqués à la FITC.

Pour le marquage à 3 couleurs, les cellules, après incubation en présence des anticorps biotinylés, sont ensuite incubées avec les anticorps monoclonaux appropriés marqués à la FITC et la PE et conjugués à la streptavidine-Tricolor (SAv-Tri, Caltag).

Le contrôle de témoin de marquage non spécifique est effectué en parallèle. L'appareil utilisé est un cytomètre de flux FACScan (Becton Dickinson, Mountain View, CA). La prise minimum d'échantillon est environ égale à 1 x 10⁴ cellules viables.

Le dosage de la production de cytokine est effectué à partir de cellules en culture sur milieu tel que décrit dans J. of Immunol., 1995, *155* (10), 4544-4550 et J. Exp. Med. 1994, *180*, 653-661. Environ 10⁵ cellules sont déposées par puits dans des cupules fond rond de microplaque 96 puits (Nunc, Roskilde, Danemark), chaque essai étant reproduit trois fois. Les cupules sont recouvertes de 10 µg/ml d'anticorps anti TCR-αβ (done H57-597) et les cellules sont incubées 48 heures en présence ou en absence d'IL-7 à 1 000 U/ml (Sanofi, Toulouse, France) pour 200 µl de volume final par puits, le surnageant est ensuite recueilli et stocké à -70°C avant dosage d'IL-4.

Le dosage d'IL-4 est effectué par ta méthode ELISA type sandwich, comme décrit dans J. of Immunol. 1995, *155* (10) 4544-4550.

Les résultats obtenus et illustrés par le tableau 1 ci-dessous, montrent que l'apparition de la sous-population de thymocytes de phénotype TCR-αβ⁺ CD44⁺, quantifiée parmi les cellules T HSA⁻CD8⁻, est retardée chez la souris NOD comparée à la souris C57BL/6 avec une diminution très significative observée à l'âge de 3 semaines.

**TABLEAU 1**

| SOUCHE | AGE (SEMAINES) | % de CD44⁻TCR-αβ⁻ dans la population de thymocytes HSA⁻CD8⁻ |
|---|---|---|
| C57BL/6 | 3 | *25.1 ± 0.8 |
| | 8 | 27.9 ± 10,1 |
| NOD | 3 | 12.5 ± 0,3 |
| | 8 | 25.3 ± 7.0 |

| | | |
|---|---|---|
| Les résultats représentent la moyenne et l'écart type obtenu sur 4 à 5 expérimentations indépendantes. * p < 0,001 versus la souris NOD âgée de 3 semaines (test de Student). | | |

Le tableau 2 ci-dessous représente les résultats obtenus sur des populations de thymocytes double négatives (CD4⁻ CD8⁻) et CD4⁺ chez les souris NOD et C57BL/6 âgées de 8 à 10 semaines. Le niveau de la sous-population CD44⁺ TCR-αβ⁺ chez la souris NOD reste encore diminué à 8 semaines comparé à la souns non atteinte de maladie auto-immune.

**TABLEAU 2**

| SOUCHE | *% de CD44⁻TCR-αβ⁻ dans | |
|---|---|---|
| | CD4⁺ | CD4⁻ CD8⁻ |
| NOD | 23 ± 8 | 34 ± 1 |
| C57BL/6 | 26 ± 10 | *59 ± 7 |

| | | |
|---|---|---|
| Moyenne et écart-type obtenus sur 3 à 4 expérimentations différentes * p < 0,001 versus de la souche NOD (test de Student) | | |

Il a été également vérifié que les cellules de thymocytes caractérisées par le phénotype HSA⁻ CD8⁻ CD44⁺ présentent bien la restriction du gène Vβ8 mentionné plus haut.

De même, il a été vérifié (voir figure 1) que les cellules thymocytes HSA⁻ CD8⁻ CD44⁺ chez la souris témoin produisaient en quantité importante de l'IL-4 lorsque stimulées par un anticorps monoclonal anti-TCR-αβ alors que chez la souris NOD, cette production chute de manière dramatique à 3 et 8 semaines, même en tenant compte du nombre réduit de cellules HSA⁻ CD8⁻ CD44⁺ (tableau 1).

### EXEMPLE 2

### Correction in vitro par l'IL-7 du déficit fonctionnel de la sous population de thymocytes chez la souris NOD.

S'il a déjà été montré que l'IL-7 induit de manière spécifique la prolifération de la sous-population de thymocytes HSA⁻ CD8⁻ CD44⁺DN (J. Exp. Med., 1994, *180*, 653-661), les résultats obtenus représentés par la figure numéro 1 montrent en particulier que, de façon surprenante, la production d'IL-4, dosée sur les thymocytes après 48 heures d'incubation en présence d'IL-7 et d'un anticorps anti-TCRαβ redevient normale chez la souris NOD adulte (âgée de 8 semaines), comparée à la souris C57BL/6 d'âge équivalent.

### EXEMPLE 3

### Expériences in vivo montrant l'effet protecteur de l'IL-7 sur la survenue du diabète chez la souris NOD.

Les deux expériences décrites ci-après montrent que les cellules lymphoïdes provenant de la glande thymique de souris NOD âgées de 3 semaines peuvent protéger contre la survenue du diabète dans un modèle de co-transfert. Précisément, 50 millions de thymocytes totaux de souns de 3 semaines injectés simultanément avec 5 à 10 millions de cellules spléniques de souris NOD diabétiques à des receveurs NOD irradiés de 10 semaines, empêchent la survenue du diabète normalement observé lorsque les cellules spléniques sont injectées seules. L'injection de moins de 10 millions de thymocytes n'a pas cet effet protecteur. Cependant, lorsque les thymocytes ont été préalablement incubés *in vitro* en présence d'IL-7 pendant 60 heures à 37°C, un million de thymocytes assure la protection dans le modèle de co-transfert décrit ci-dessus.

En outre, il est connu qu'une injection de cyclophosphamide (200 mg/kg) provoquait l'apparition d'un diabète chez des souris NOD mâles âgées de 8 semaines dans un délai très inférieur (10 à 20 jours) à celui de l'apparition spontanée du diabète (2 à 4 mois). Un traitement *in vivo* par l'IL-7 consistant en 2 injections d'IL-7, administrées la veille et le lendemain de l'injection de cyclophosphamide protège contre la survenue du diabète induit par la cyclophosphamide.

Ces deux séries d'expériences indiquent que l'IL-7 a un rôle protecteur contre la survenue d'un diabète chez les souris génétiquement prédisposées.

### 3.1 Protection du diabète par des thymocytes incubés en présence d'interleukine-7 dans un modèle de co-transfert.

Des thymocytes sont préparés à partir d'un thymus de souris NOD femelles âgées de 3 semaines. Ces thymocytes sont incubés *in vitro* pendant 60 à 72 heures à 37°C dans du milieu RPMI contenant 10 % de sérum de veau foetal et additionné d'IL-7 (500-1000 U/ml). Au terme de cette incubation, on injecte simultanément 4, 2 ou 1 million(s) de ces thymocytes et 5 ou 10 millions de cellules spléniques de souris NOD récemment devenues diabétiques à des souns NOD mâles âgées de 10 semaines irradiées par une dose de 700 rad. La survenue d'un diabète est suivie trois fois par semaine par la recherche d'une glycosurie et confirmée lorsqu'une glycosurie est observée par la mise en évidence d'une hyperglycémie.

Les résultats indiqués dans le tableau 3 ci-joint montrent que les thymocytes incubés en présence d'IL-7 protègent contre le diabète à des doses qui ne sont pas protectrices lorsque des thymocytes non incubés en présence d'IL-7 sont utilisés.

**TABLEAU 3**

| Effet protecteur de thymocytes totaux traités par l'IL-7 dans un modèle de diabète induit par transfert passif de cellules provenant de souris diabétiques. | | | | | | |
|---|---|---|---|---|---|---|
| Expérience | Thymocytes transférés | Nbre d'animaux | Nbre de souns diabétiques en fonction du nombre de semaines après le transfert | | | |
| | | | 1 sem. | 4 sem. | 6 sem. | 8 sem. |
| No 1 | - | 6 | 0 | 4 | 5 | 5 |
| | non traités par l'IL-7 | 6 | 0 | 3 | 4 | 6 |
| | | 6 | 0 | 0 | 0 | 1 |
| No 2 | - | 5 | 0 | 0 | 4 | 4 |
| | traités par l'IL-7 | 8 | 0 | 0 | 0 | 0 |
| No 3 | - | 8 | 0 | 6 | 6 | NT |
| | traités par l'IL-7 | 6 | 0 | 0 | 0 | NT |
| NT : non testé | | | | | | |

### 3.2. Protection contre le diabète induit par une injection de cyclophosphamide par des injections d'interleukine-7

Des souris NOD femelles âgées de 6 à 7 semaines ont reçu une injection de 200 ma/kg de cyclophosphamide, encadrée aux jours -1 et +1 par une injection intraveineuse de 1 µg d'IL-7. La survenus d'un diabète est détectée par la recherche d'une glycosurie et d'une hyperglycémie selon les modalités indiquées dans l'exemple 1.

Les résultats montrés dans le tableau 4 indiquent que le traitement par l'IL-7 prévient significativement la survenue du diabète par rapport aux souris ayant reçu du cyclophosphamide sans IL-7.

**TABLEAU 4**

| Effet protecteur de l'IL-7 dans le modèle de diabète induit par le cyclophosphamide | | |
|---|---|---|
| Traitement | Nombre de souris | Nombre de souris diabétiques après 2 semaines suivant l'injection de cyclophosphamide |
| - | 10 | 8 |
| IL-7 | 10 | 2 |

L'ensemble de ces résultats montre que les souns NOD présentent de manière précoce un déficit d'une sous-population de cellules thymocytaires HSA⁻CD8TCR-αβ⁺, exprimant le marqueur CD44, avec la restriction Vβ8 et ayant la capacité de produire l'IL-4 (appelées cellules T NK1⁺, récemment identifiée dans les souches non-autoimmunes). Ce déficit numérique est associé de manière surprenante à un déficit fonctionnel exprimé par une réduction importante de la production d'IL-4.

Les résultats indiquent que l'anomalie de ce déficit chez les souris NOD, touchant à la fois les sous-populations de thymocytes CD4⁻CD8⁻ (ON) et CD4⁺ (simple positive) est corrigée *in vitro* par l'utilisation de l'IL-7.

D'autre part, les expériences menées *in vivo*, que ce soit par injection de cellules préalablement incubées en présence d'IL-7 ou par injection directe d'une quantité efficace d'IL-7 chez la souris montrent que l'utilisation d'IL-7 a un effet protecteur contre le déclenchement de maladie auto-immune comme en particulier le diabète.

### EXEMPLE 4

### Traitement in vivo par l'IL-7

Le traitement *in vivo* par l'IL-7 potentialise la production d'IL-4 par les cellules T spléniques (figure 2). Cet effet de l'IL-7 est obtenu dans l'ensemble des souches de souris testées (C57BL/6, BALB/c, NOD) et il est maximal chez la souris auto-immune NOD qui présente un déficit de production d'IL-4 en périphérie (légende de la figure 2). L'absence d'augmentation de la production d'IL-4 chez les souris C57BL/6 dépourvues du gène de la β2-microglobuline et donc déficientes en cellules T NK1⁺, suggère que l'action de de l'IL-7 est ciblée sur les celules T NK1⁺.

Le protocole d'injection d'IL-7 par voie sous cutanée comprend deux doses de 2µg par jour pendant une durée de 4 à 7 jours consécutifs. Des souris contrôles sont traitées par un volume identique de solution d'excipient (albumine sérique bovine). La production d'IL-4 est relevée après une unique injection (1,33µg) par voie intraveineuse d'un anticorps dirigé spécifiquement contre la molécule CD3 murine (anti-CD3, clone 145-2C11, IgG de hamster). Les animaux sont sacrifiés 90 minutes suivant l'injection de l'anticorps anti-CD3 ; les rates sont immédiatement prélevées et les cellules spléniques mises en culture pendant 90 minutes en l'absence de toute stimulation (Yoshimoto *et al*., J. Exp. Med, *179*, 1285-1295). Le milieu RPMI contenant du sérum de veau foetal décomplémenté (10%), du β-mercaptoéthanol (0,05 mM) et des antibiotiques (pénicilline 100 Ul/ml et streptomycine 100µg/ml) est utilisé pour l'ensemble des expériences de culture cellulaire. Les sumageants sont prélevés et leur contenu en IL-4 déterminé par le test biologique CT.4S, une lignée dépendant de l'IL-4 (Hu-Li *et al*., J. Immunol., *142*, 800-807). La concentration d'IL-4 est exprimée en U/ml (une unité correspond approximativement à 1 pg) d'après une courbe standard établie avec des dilutions sériées d'IL-4 recombinante murine. La limite de sensibilité du test est d'environ 10 U/ml.

L'absence de production d'IL-4 en périphérie par des cellules T NK1⁺ chez la souris NOD pourrait expliquer le déficit de la fonction Th2 qui s'accompagne de l'émergence de cellules diabétogènes de type Th1 responsables de la maladie auto-immune dans cette souche.

Ces résultats qui constituent la démonstration princeps d'un effet *in vivo* pharmacologique de l'IL-7 sur la production d'IL-4 par les cellules T NK1⁺ ouvrent des perspectives sur le rôle protecteur de l'IL-7 vis à vis de l'apparition du diabète auto-immun. Plus généralement, ces données expérimentales permettent de proposer l'utilisation de l'IL-7 comme arme thérapeutique de déviation de la réponse immune en faveur d'un profil de type Th2.

## Revendications

1. Utilisation de l'interleukine-7 ou de lymphocytes T préalablement incubés en présence d'IL-7, pour la préparation d'un médicament ou d'une composition pharmaceutique pour le traitement d'une maladie auto-immune.

2. Utilisation selon la revendication 1, ladite maladie auto-immune étant générée par un défaut de production d'IL-4 par les cellules Th2.

3. Utilisation selon la revendication 1 ou 2, ladite maladie auto-immune étant générée par un défaut de production d'IL-4 lié à un déficit quantitatif et fonctionnel de cellules T de sous type HSA⁻, CD4⁻CD8⁻ ou CD4⁺CD8⁻, CD44⁺, TCR-αβ⁺, Vβ8⁺, NK1.1⁺.

4. Utilisation selon la revendication 1, 2 ou 3, ladite maladie auto-immune étant choisie parmi le diabète mellitus insulinodépendant, l'encéphalo-myélite auto-immune, l'arthrite rhumatoïde auto-immune, la polyarthrite, les hépatites auto-immunes de type 2, la gastrite auto-immune, la sclérose auto-immmune, la sialadénite, l'adrénalite, l'oophorite, les glomérulonéphrites, la thyroïdite auto-immune, et les mécanismes pathogéniques de type auto-immun dans une thérapie associée au traitement du SIDA.

5. Utilisation selon la revendication 1, 2, 3 ou 4, ladite maladie auto-immune étant le diabète mellitus insulinodépendant.

6. Utilisation selon l'une quelconque des revendications précédentes, lesdits lymphocytes T étant des cellules autologues ou syngéniques des cellules du patient auquel la composition pharmaceutique est destinée.

7. Procédé de fabrication d'une composition pharmaceutique pour le traitement des maladies auto-immunes, comprenant le mélange des lymphocytes T autologues ou syngéniques des cellules du patient auquel la composition est destinée, lesdits lymphocytes T ayant été préalablement incubés en présence d'IL-7, avec un véhicule ou diluant pharmaceutiquement acceptable, éventuellement en association avec d'autres principes actifs.

8. Procédé de fabrication d'une composition pharmaceutique pour le traitement d'une maladie auto-immune selon la revendication 7, ladite maladie auto-immune étant générée par un défaut de production d'IL-4 par les cellules Th2.

9. Procédé de fabrication d'une composition pharmaceutique pour le traitement d'une maladie auto-immune selon la revendication 7 ou 8, ladite maladie auto-immune étant générée par un défaut de production d'IL-4 lié à un déficit quantitatif et fonctionnel de cellules T de sous-type HSA⁻, CD4⁻CD8⁻ ou CD4⁺CD8⁻, CD44⁺, TCR-αβ⁺, Vβ8⁺, NK1.1⁺.

10. Procédé de fabrication d'une composition pharmaceutique pour le traitement d'une maladie auto-immune selon la revendication 7, 8 ou 9, ladite maladie auto-immune étant le diabète mellitus insulinodépendant.

## Claims

1. Use of interleukin-7 or T lymphocytes which have previously been incubated in the presence of IL-7, for preparing a medicament or a pharmaceutical composition for treating an autoimmune disease.

2. Use according to claim 1, said autoimmune disease being caused by a fault in the production of IL-4 by the Th2 cells.

3. Use according to claim 1 or 2, said autoimmune disease being caused by a fault in the production of IL-4 accompanied by a quantitative and functional deficit of T cells of subtype HSA⁻,CD4⁻CD8⁻ or CD4⁺CD8⁻, CD44⁺, TCR-αβ⁺, Vβ8⁺, NK1.1⁺.

4. Use according to claim 1, 2 or 3, said autoimmune disease being selected from among insulin-dependent diabetes mellitus, autoimmune encephalomyelitis, autoimmune rheumatoid arthritis, polyarthritis, autoimmune type 2 hepatitis, autoimmune gastritis, autoimmune sclerosis, sialadenitis, adrenalitis, oophoritis, glomerulonephritis, autoimmune thyroiditis and the pathogenic mechanisms of the autoimmune type in combined therapy for treating AIDS.

5. Use according to claim 1, 2, 3 or 4, said autoimmune disease being insulin-dependent diabetes mellitus.

6. Use according to any one of the'preceding claims, said T lymphocytes being cells which are autologous or syngenic with the cells of the patient for whom the pharmaceutical composition is intended.

7. Process for producing a pharmaceutical composition for treating autoimmune diseases, comprising mixing the T lymphocytes which are autologous or syngenic with the cells of the patient for whom the composition is intended, said T lymphocytes having previously been incubated in the presence of IL-7, with a pharmaceutically acceptable carrier or diluent, optionally combined with other active ingredients.

8. Process for producing a pharmaceutical composition for treating an autoimmune disease according to claim 7, said autoimmune disease being caused by a fault in the production of IL-4 by the Th2 cells.

9. Process for producing a pharmaceutical composition for treating an autoimmune disease according to claim 7 or 8, said autoimmune disease being caused by a fault in the production of IL-4 accompanied by a quantitative and functional deficit of T cells of subtype HSA⁻, CD4⁻CD8⁻ or CD4⁺CD8⁻, CD44⁺, TCR-αβ⁺, Vβ8⁺, NK1.1⁺.

10. Process for producing a pharmaceutical composition for treating an autoimmune disease according to claim 7, 8 or 9, said autoimmune disease being insulin-dependent diabetes mellitus.

## Patentansprüche

1. Verwendung von Interleukin-7 oder von T-Lymphozyten, die zuvor in Gegenwart von IL-7 inkubiert worden sind, für die Herstellung eines Arzneimittels oder einer pharmazeutischen Zubereitung zur Behandlung einer Autoimmunkrankheit.

2. Verwendung nach Anspruch 1, wobei die Autoimmunkrankheit durch einen Fehler der Bildung von IL-4 durch die Th2-Zellen verursacht ist.

3. Verwendung nach Anspruch 1 oder 2, wobei die Autoimmunkrankheit durch einen Fehler der Bildung von IL-4 verursacht ist, die mit einem quantitativen und funktionellen Defizit von T-Zellen des Untertyps HSA⁻, CD4⁻CD8⁻ oder CD4⁺CD8⁻, CD44⁺, TCR-αβ⁺, Vβ8⁺, NK1.1⁺ verknüpft ist.

4. Verwendung nach Anspruch 1, 2 oder 3, wobei die Autoimmunkrankheit ausgewählt ist aus insulinabhängigem Diabetes mellitus, autoimmuner Encephalomyelitis, autoimmuner rheumatoider Arthritis, Polyarthritis, Autoimmun-Hepatitis Typ 2, autoimmuner Gastritis, autoimmuner Sklerose, Sialadenitis, Adrenalitis, Oophoritis, Glomerulonephritis, autoimmuner Thyroiditis und pathogenen Mechanismen vom Autoimmun-Typ bei einer mit der Behandlung von AIDS verknüpften Therapie.

5. Verwendung nach Anspruch 1, 2, 3 oder 4, wobei die Autoimmunkrankheit insulinabhängiger Diabetes mellitus ist.

6. Verwendung nach einem der vorhergehenden Ansprüche, wobei die T-Lymphozyten autologe oder syngene Zellen von Zellen des Patienten sind, für den die pharmazeutische Zubereitung bestimmt ist.

7. Verfahren zur Herstellung einer pharmazeutischen Zubereitung zur Behandlung von Autoimmunkrankheiten, umfassend das Vermischen von autologen oder syngenen T-Lymphozyten von Zellen des Patienten, für den die Zubereitung bestimmt ist, wobei die T-Lymphozyten zuvor in Gegenwart von IL-7 inkubiert worden sind, mit einem pharmazeutisch annehmbaren Trägermaterial oder Verdünnungsmittel, gegebenenfalls in Kombination mit anderen Wirkstoffen.

8. Verfahren zur Herstellung einer pharmazeutischen Zubereitung zur Behandlung einer Autoimmunkrankheit nach Anspruch 7, welche Autoimmunkrankheit durch einen Fehler der Bildung von IL-4 durch die Th2-Zellen verursacht ist.

9. Verfahren zur Herstellung einer pharmazeutischen Zubereitung zur Behandlung einer Autoimmunkrankheit nach Anspruch 7 oder 8, wobei die Autoimmunkrankheit durch einen Fehler der Produktion von IL-4 verursacht ist, die mit einem quantitativen und funktionellen Defizit von T-Zellen des Untertyps HSA⁻, CD4⁻CD8⁻ oder CD4⁺CD8⁻, CD44⁺, TCR-αβ⁺, Vβ8⁺, NK1.1⁺ verknüpft ist.

10. Verfahren zur Herstellung einer pharmazeutischen Zubereitung zur Behandlung einer Autoimmunkrankheit nach Anspruch 7, 8 oder 9, wobei die Autoimmunkrankheit insulinabhängiger Diabetes mellitus ist.
